# FASCICULE DE BREVET EUROPEEN

(11) **EP 3 841 089 B1**
(45) Date de publication et mention de la délivrance du brevet: **12.10.2022**
(21) Numéro de dépôt: 19753104.9
(22) Date de dépôt: 19.08.2019
(51) Int. Cl.: C07C 403/14, C07C 45/28, C07C 45/65, C07C 47/21

(54) **PROCÉDÉ DE SYNTHÈSE DE LA VITAMINE A**
VERFAHREN ZUR SYNTHESE VON VITAMIN A
METHOD FOR SYNTHESISING VITAMIN A

(30) Priorité: 20.08.2018 FR 1857548
(43) Date de publication de la demande: 30.06.2021
(73) Titulaire: Adisseo France S.A.S., 92160 Antony (FR)
(72) Inventeur: REY, Patrick, 69003 Lyon (FR); HUET, Robert, 75015 Paris (FR); AUBRY, Sylvain, 62100 Calais (FR); HENRYON, Vivien, 69007 Lyon (FR)
(74) Mandataire: Germain Maureau
(86) Numéro de dépôt international: PCT/EP2019/072083
(87) Numéro de publication internationale: WO 2020/038857

(56) Documents cités:
- ALAIN VALLA ET AL: "New Syntheses of Retinal and Its Acyclic Analog[gamma]-Retinal by an Extended Aldol Reaction with a C6 Building Block That Incorporates a C5 Unit after Decarboxylation. A Formal Route to Lycopene and&bgr;-Carotene", HELVETICA CHIMICA ACTA, vol. 90, no. 3, 1 mars 2007 (2007-03-01), pages 512-520, XP055143601, ISSN: 0018-019X, DOI: 10.1002/hlca.200790053 cité dans la demande

## Description

La présente invention concerne de nouvelles réactions de transformation de composés sesquiterpéniques permettant d'ouvrir une nouvelle voie d'accès à la vitamine A (C₂₀H₃₀O), ses précurseurs et ses dérivés.

La synthèse à l'échelle industrielle de la vitamine A est réalisée par diverses méthodes classiques. Entre autres préparations, la vitamine A peut être obtenue par la voie de condensation dite C15 + C5, comme par exemple la réaction dite de Julia qui implique la chimie des sulfones, selon laquelle le vinyl-β-ionol est traité par un anion phénylsulfinate pour conduire à une sulfone en C15 à laquelle est ajouté un bromure d'allyle pour obtenir une sulfone en C20. Celle-ci est ensuite convertie, par élimination, en acétate de vitamine A qui est généralement utilisé en l'état, la vitamine A étant très instable, ou alors saponifié en vitamine A.

D'autres procédés sont aussi utilisés. Ainsi, la vitamine A peut être fabriquée par un procédé de couplage en C15 + C5 via une réaction de Wittig ; ce procédé met toutefois en œuvre des intermédiaires à effets cancérogènes, mutagènes ou toxiques pour la reproduction (CMR), tel que l'acétate en C5, et nécessite une unité de régénération de la phosphine au phosgène qui est un gaz très toxique.

Une autre voie d'accès réside dans un procédé de couplage en C6 + C14 au travers de la chimie des alcynes ; elle présente les inconvénients d'engager certaines matières premières, comme l'acétylène, le nButyl-Li, qui présentent des risques HSE (Hygiène - Sécurité - Environnement) évidents et d'impliquer des intermédiaires époxydes peu stables et toxiques.

Dans le document A Valla et al., Helvetica Chimica Acta, vol. 90 (2007) 512-520 concernant la préparation du rétinal, la forme aldéhyde de la vitamine A, les auteurs décrivent la synthèse en deux étapes du déhydro-cyclofarnésal à partir de la β-ionone. Cette synthèse passe par un intermédiaire nitrile en C15 qui est réduit par l'hydrure de diisobutylaluminium en déhydro-cyclofarnésal.

Ces procédés de synthèse n'ont pourtant pas connu de véritables évolutions depuis leur découverte, et à ce jour, il est important de recourir à de nouveaux procédés industriels de synthèse plus sûrs et plus économiques.

L'invention fournit une nouvelle voie de synthèse de la vitamine A, à partir de sesquiterpènes, et en particulier le farnésène, le farnésal et le farnésol, ou de dérivés sesquiterpéniques, et en particulier le nérolidol et le déhydronérolidol. Ces composés sont présents dans la nature, on les trouve dans certaines essences végétales et dans ou à la surface de certains fruits, desquels ils peuvent être extraits, ils sont aussi biosynthétisés par des microorganismes, notamment des champignons. La ressource en réactif étant donc inépuisable, l'invention apporte une solution pérenne aux problèmes de coût des procédés classiques et contribue à un réel progrès dans la fabrication de la vitamine A, mais aussi celle d'intermédiaires pour d'autres synthèses.

Parmi les réactions objets de la présente invention, l'une constitue une étape clé dans la synthèse de la vitamine A. Il s'agit d'un procédé de préparation du déhydro-cyclofarnésal à partir de déhydro-farnésal par cyclisation en présente d'un acide.

La réaction de conversion du déhydro-farnésal en déhydro-cyclofarnésal selon l'invention constitue une étape importante dans la chaîne de synthèse de la vitamine A, de ses précurseurs ou de ses dérivés, en ce qu'elle permet accéder à une structure en C15 contenant le cycle de la vitamine A sans recourir au vinyl-β-ionol.

Le déhydro-farnésal peut être obtenu par déshydrogénation du farnésal selon une réaction qui est aussi un objet de l'invention, ce qui est avantageux car le farnésal est un réactif facilement accessible. Il peut en effet être produit synthétiquement à partir de farnésène, de farnésol, de farnésoate d'éthyle, de nérolidol ou de déhydro-nérolidol selon des procédés connus de l'homme du métier ((Tetrahedron Letters 2016, 57, 40, 4496-4499 ; New Journal of Chemistry 2001, 25, 7, 917-929 ; Catal. Comm. 2014, 44, 40-45), mais il est aussi être isolé d'huiles essentielles, comme celles de lemongrass.

Comme indiqué ci-dessus, le farnésal peut être fabriqué à partir du farnésène, et il peut en particulier être préparé par oxydation du farnésène selon un procédé qui est encore un autre objet de l'invention.

Avant d'aborder l'invention plus en détails, les définitions de termes employés dans le présent texte sont ci-après données.

Toute référence à un composé insaturé s'étend aux isomères de ce composé, en particulier à ses régioisomères et ses stéréoisomères.

A titre d'exemple, le terme farnésène inclut les régioisomères alpha et beta du farnésène, ainsi que les stéréoisomères de chacun d'eux, comme illustré ci-dessous :
- L'a-farnésène (3,7,11-triméthyl-1,3,6,10-dodécatetraène) ayant la formule suivante 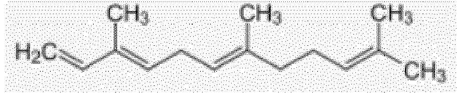 qui peut exister sous la forme des 4 isomères suivants (3E, 6E), (3E, 6Z), (3Z, 6Z) et (3Z, 6E), et
- le β-farnésène (7,11-diméthyl-3-méthylène-1,6,10-dodécatriène) ayant la formule suivante 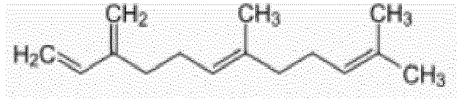
qui peut exister sous la forme des 2 isomères suivants (6E) et (6Z).

A titre d'un autre exemple, le terme cyclofarnésal couvre les deux régioisomères suivants :
- le cyclofarnésal de formule : 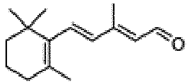 et ses isomères E, Z
- l'énol de cyclofarnésal de formule : 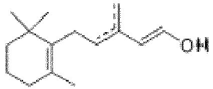 et ses isomères E, Z.

Cette définition s'applique notamment au farnésal, au déhydro-farnésal, au déhydro-cyclofarnésal, au farnésol, au nérolidol, au déhydro-nérolidol, au rétinal, dont les appellations couvrent tous leurs isomères respectifs.

Par acide, on entend tout composé minéral ou organique, sous toute forme notamment liquide, solide ou gazeux, présent dans le milieu de réaction en phase homogène ou hétérogène, et possédant une lacune électronique le rendant susceptible d'accepter un doublet d'électrons.

L'invention est ci-après exposée plus en détails.

L'invention concerne ainsi la cyclisation du déhydro-farnésal en déhydro-cyclofarnésal effectuée en présence d'un acide. Avantageusement, l'acide est choisi parmi les acides de Lewis, les acides de Bronstedt et les zéolithes, et toutes combinaisons de ceux-ci. A titre d'exemples, il peut être l'acide chlorosulfonique, le triflurorure de bore ou un de ses éthérate, ou encore le chlorure d'étain.

L'invention concerne aussi la fabrication de déhydro-farnésal à partir de farnésal par une déshydrogénation catalytique. Selon des conditions préférées, cette déshydrogénation est réalisée en présence d'un ou de sels de palladium(II). On utilisera avantageusement des sels de type Pd(OAc)₂, une base et un agent oxydant. Typiquement, les conditions de cette réaction sont les suivantes : Pd(OAc)₂, une base choisie parmi Na₂CO₃ et K₂CO₃, et en présence d'oxygène. L'utilisation de solvants de type polaire protique est plus particulièrement indiquée avec par exemple l'emploi de diméthylsulfoxyde (DMSO), N-méthylpyrrolidone (NMP) ou diméthylol-éthylène urée (DMEU). Ces conditions peuvent être complétées par la présence de ligands notamment choisis parmi des composés aromatiques comme l'hydroquinone, le catéchol ou le diazafluorenone, ou d'additifs précurseurs de pi-allyl comme le diéthylphosphate d'allyle.

L'invention apporte aussi un procédé d'oxydation du farnésène en farnésal. Cette réaction est effectuée dans les conditions catalytiques d'un procédé de type Wacker en présence d'au moins un métal précieux, principalement le palladium. Avantageusement, le milieu réactionnel comprend des sels de palladium(II) tels que PdCl₂, des sels de cuivre et un agent oxydant. A titre d'exemple, la réaction est conduite en présence de PdCl₂(CH₂CH₂), CuCl₂ - LiMoO₄.

L'invention concerne aussi la fabrication de déhydro-farnésal à partir de farnésène, selon un procédé mettant en œuvre les étapes précitées, à savoir :
oxydation du farnésène en farnésal, puis
déshydrogénation du farnésal en déhydro-farnésal,
ces deux étapes étant effectuées dans les conditions de l'invention décrites ci-dessus.

Un autre des objets de l'invention est un procédé de synthèse de la vitamine A à partir de farnésène, qui comprend au moins l'une des étapes précédemment décrites, à savoir :
- la cyclisation du déhydro-farnésal en déhydro-farnésal ;
- l'oxydation du farnésène en farnésal ;
- la déshydrogénation du farnésal en déhydro-farnésal.

Avantageusement, le procédé de synthèse de la vitamine A comprend au moins deux, voire l'ensemble de ces étapes, et est suivi d'une conversion du déhydro-cyclofarnésal en vitamine A, selon un procédé à la portée de l'homme du métier ayant un niveau moyen de connaissances. Selon une variante avantageuse de l'invention, la vitamine A est obtenue par réaction du déhydro-cyclofarnésal avec un éther d'énol silylé du prénal, par exemple un éther d'énol triméthylsilylé, conduisant au rétinal.

L'invention est ci-après illustrée dans des exemples réalisés dans des conditions réactionnelles qui viennent supporter la mise en pratique de l'invention mais auxquelles elle n'est certainement pas restreinte.

Dans les exemples, les abréviations employées sont ci-après définies :
TT définit le taux de transformation ;
RR définit un rendement sur réactif ;
RR dosé définit un rendement sur réactif dosé dans un milieu réactionnel.

### Exemple 1 : Cyclisation du déhydro-farnésal en déhydro-β-cyclofarnésal

La cyclisation du déhydro-farnésal en déhydro-β-cyclofarnésal est effectuée selon le schéma ci-dessous :

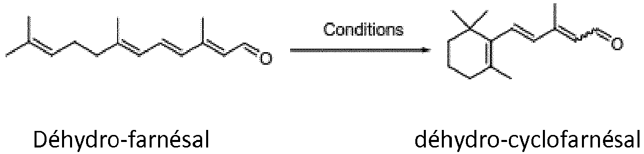

Les conditions opératoires sont les suivantes :
Dans un vial de 4 mL muni d'un barreau aimanté, et placé sous atmosphère d'azote sont introduits le déhydro-farnésal (0,115 mmol), le solvant (0,46 mL) et finalement la source d'acide, ajoutée à la température indiquée. Le milieu réactionnel est ensuite agité sous agitation magnétique à la température spécifiée. Les temps indiqués dans le tableau correspondent aux meilleurs rendements obtenus. Les prélèvements (200 µL) sont analysés par chromatographie gazeuse (GC). Différentes conditions ont été testées, les plus représentatives sont indiquées dans le tableau 1.

**Tableau 1**

| Conditions | TT_{déhyfrofarnésal} (GC, %) | RR dosé_{déhydro-β-cyclofarnésal} (GC, %) |
|---|---|---|
| ClSO₃H, CH₂Cl₂, -78°C, 30 min. | 94 | 22 |
| SnCl₄ (2 éq.), toluène, 0°C, 30 min. | 100 | 35 |
| BF₃.Et₂O (2 éq.), toluène, 30°C | 100 | 14 |

### Exemple 2 : Oxydation du β-farnésène en farnésal

L'oxydation du β-farnésène en farnésal est effectuée selon le schéma ci-dessous :

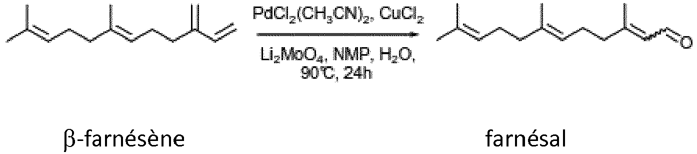

Les conditions opératoires sont les suivantes :
Dans un vial de 4 mL muni d'un barreau aimanté, et placé sous atmosphère d'azote sont introduits le PdCl₂(CH₃CN)₂ (0,25 mmol), Li₂MoO₄ (1,7 mmol), CuCl₂ (0,3 mmol), le solvant (2 mL), H₂O (0,2 mL) et l'alcène (1,0 mmol). Le milieu réactionnel est ensuite agité sous agitation magnétique à 90°C. Les temps indiqués dans le tableau ci-dessous correspondent aux meilleurs rendements obtenus. Les prélèvements (200 µL) sont analysés par GC.

**Tableau 2**

| Conditions | Solvant | TT_{farnésène}(GC, %) | RR dosé_{farnésal} (GC, %) |
|---|---|---|---|
| β-farnésène, 24h, 90°C | NMP | 73 | 17 |
| | DMEU | 75 | 13 |
| | DMSO | 57 | 6 |

### Exemple 3 : Déshydrogénation du farnésal en déhydro-farnésal

La déshydrogénation du farnésal en déhydro-farnésal est effectuée selon le schéma ci-dessous :

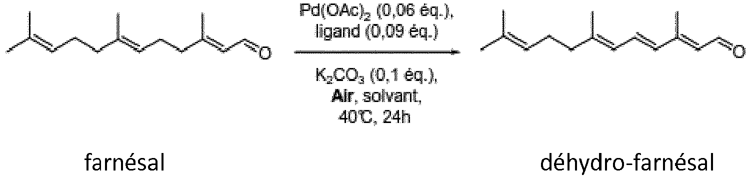

Les conditions opératoires sont les suivantes :
Dans un vial ouvert de 4 mL muni d'un barreau aimanté, et placé sous atmosphère d'azote sont introduits le Pd(OAc)₂ (0,03 mmol), K₂CO₃ (0,05 mmol), la 4,5-diazafluorénone (DAF) (0.045 mmol), le solvant (0,46 mL) et le farnésal (0,5 mmol). Le milieu réactionnel est ensuite agité sous agitation magnétique à 30°C. Les prélèvements (environ 200 µL) sont réalisés après 1h, 3h, 5h, 7h, 24h puis analysés par GC.
Ces conditions conduisent à un TT_{farnésal} (GC, %) de 92% et RR dosé_{déhydrofarnésal} (GC, %) de 60%.

### Exemple 4 : Préparation du rétinal à partir du déhydro-cyclofarnésal

Le rétinal est préparé selon le schéma ci-dessous :

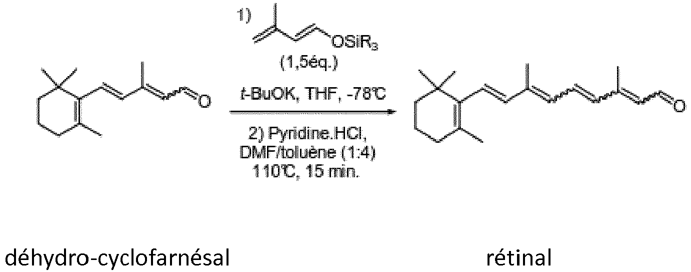

En détails, cette transformation a lieu dans les conditions suivantes, en passant par des intermédiaires non isolés :

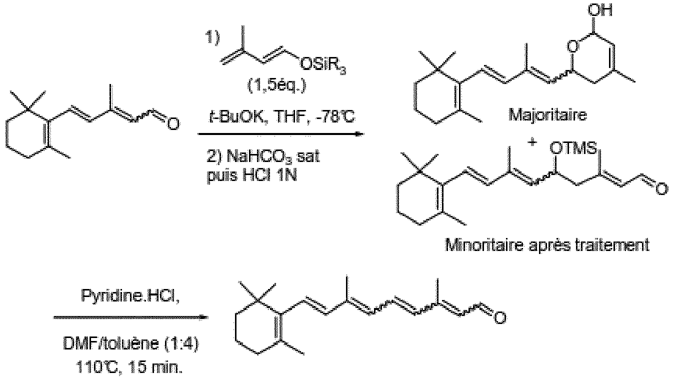

Ces conditions conduisent à un TT_{déhydro-β-cyclofarnésal} (CCM) de 100% et RR dosé_{rétinal} de 40%.

## Revendications

1. Procédé de préparation du déhydro-cyclofarnésal à partir de déhydro-farnésal par cyclisation en présente d'un acide.

2. Procédé selon la revendication 1, **caractérisé en ce que** la cyclisation est réalisée en présence d'un acide choisi parmi les acides de Lewis, les acides de Bronstedt et les zéolithes.

3. Procédé selon la revendication 2, **caractérisé en ce que** l'acide est choisi parmi l'acide chlorosulfonique, le triflurorure de bore et ses éthérates, et le chlorure d'étain.

4. Procédé selon l'une quelconque des revendications 1 à 3, **caractérisé en ce que** le déhydro-farnésal est obtenu à partir du farnésal par déshydrogénation.

5. Procédé selon la revendication 4, **caractérisé en ce que** la déshydrogénation du farnésal est réalisée en présence d'au moins un sel de palladium(II).

6. Procédé selon la revendication 4 ou 5, **caractérisé en ce que** le farnésal est obtenu à partir de farnésène, du farnésol, du farnésoate d'éthyle, du nérolidol ou du déhydro-nérolidol.

7. Procédé selon l'une quelconque des revendications 4 à 6, **caractérisé en ce que** le déhydro-farnésal est obtenu à partir du farnésène, et **en ce qu'**il comprend les étapes suivantes :
oxydation du farnésène en farnésal , puis
déshydrogénation du farnésal en déhydro-farnésal.

8. Procédé selon la revendication 7, **caractérisé en ce que** l'oxydation du farnésène est réalisée dans les conditions catalytiques de type Wacker en présence d'au moins un métal précieux tel que le palladium.

9. Procédé de synthèse de la vitamine A à partir de farnésène, **caractérisé en ce qu'**il comprend au moins un procédé selon l'une quelconque des revendications 1 à 8, puis la conversion du déhydro-cyclofarnésal en vitamine A.

10. Procédé de synthèse de la vitamine A selon la revendication 9, **caractérisé en ce que** le déhydro-cyclofarnésal est converti en vitamine A par réaction avec un éther d'énol silylé du prénal.

## Patentansprüche

1. Verfahren zur Herstellung von Dehydrocyclofarnesal aus Dehydrofarnesal durch Zyklisieren in Anwesenheit einer Säure.

2. Verfahren nach Anspruch 1, **dadurch gekennzeichnet, dass** die Zyklisierung in Anwesenheit einer Säure durchgeführt wird, die aus den LewisSäuren, den Bronstedt-Säuren und den Zeolithen ausgewählt ist.

3. Verfahren nach Anspruch 2, **dadurch gekennzeichnet, dass** die Säure aus der Chlorsulfonsäure, Bortrifluorid und seinen Etheraten und Zinnchlorid ausgewählt ist.

4. Verfahren nach einem der Ansprüche 1 bis 3, **dadurch gekennzeichnet, dass** das Dehydrofarnesal aus Farnesal durch Dehydrieren gewonnen wird.

5. Verfahren nach Anspruch 4, **dadurch gekennzeichnet, dass** die Dehydrierung des Farnesals in Anwesenheit von mindestens einem Palladium-(II)-Salz durchgeführt wird.

6. Verfahren nach Anspruch 4 oder 5, **dadurch gekennzeichnet, dass** das Farnesal aus Farnesen, Farnesol, Ethylfarnesoat, Nerolidol oder Dehydronerolidol gewonnen wird.

7. Verfahren nach einem der Ansprüche 4 bis 6, **dadurch gekennzeichnet, dass** das Dehydrofarnesal aus Farnesen gewonnen wird und dass es die folgenden Schritte umfasst:
Oxydiren des Farnesens in Farnesal, dann
Dehydrieren des Farnesals in Dehydrofarnesal.

8. Verfahren nach Anspruch 7, **dadurch gekennzeichnet, dass** die Oxydation des Farnesens unter katalytischen Bedingungen vom Wacker-Typ in Anwesenheit von mindestens einem Edelmetall wie Palladium durchgeführt wird.

9. Verfahren zur Synthese von Vitamin A aus Farnesen, **dadurch gekennzeichnet, dass** es mindestens ein Verfahren nach einem der Ansprüche 1 bis 8, dann die Umwandlung von Dehydrocyclofarnesal in Vitamin A umfasst.

10. Verfahren zur Synthese von Vitamin A nach Anspruch 9, **dadurch gekennzeichnet, dass** das Dehydrocyclofarnesal durch die Reaktion mit einem silysierten Prenal-Enolether in Vitamin A umgewandelt wird.

## Claims

1. A method for preparing dehydro-cyclofarnesal from dehydro-farnesal by cyclization in the presence of an acid.

2. The method according to claim 1, **characterized in that** the cyclization is carried out in the presence of an acid selected from Lewis acids, Bronsted acids and zeolites.

3. The method according to claim 2, **characterized in that** the acid is selected from chlorosulfonic acid, boron trifluoride and its etherates, and tin chloride.

4. The method according to any one of claims 1 to 3, **characterized in that** the dehydro-farnesal is obtained from farnesal by dehydrogenation.

5. The method according to claim 4, **characterized in that** the dehydrogenation of farnesal is carried out in the presence of at least one palladium(II) salt.

6. The method according to claim 4 or 5, **characterized in that** the farnesal is obtained from farnesene, farnesol, ethyl farnesoate, nerolidol or dehydro-nerolidol.

7. The method according to any one of claims 4 to 6, **characterized in that** the dehydro-farnesal is obtained from farnesene, and **in that** it comprises the following steps:
oxidation of farnesene into farnesal, then
dehydrogenation of farnesal in dehydro-farnesal.

8. The method according to claim 7, **characterized in that** the oxidation of farnesene is carried out under Wacker-type catalytic conditions in the presence of at least one precious metal such as palladium.

9. A method for the synthesis of vitamin A from farnesene, **characterized in that** it comprises at least one method according to any one of claims 1 to 8, then the conversion of dehydro-cyclofarnesal into vitamin A.

10. The method for the synthesis of vitamin A according to claim 9, **characterized in that** the dehydro-cyclofarnesal is converted into vitamin A by reaction with a silylated enol ether of prenal
